# EUROPÄISCHE PATENTANMELDUNG

(11) **EP 2 042 185 A1**
(43) Veröffentlichungstag der Anmeldung: **01.04.2009**
(21) Anmeldenummer: 08105029.6
(22) Anmeldetag: 13.08.2008
(51) Int. Cl.: A61K 38/00, A61K 39/00, C12N 15/00

(54) **Zwei Wirkkomponenten umfassendes Arzneimittel zur gezielten Behandlung eines Karzinoms**

(30) Priorität: 03.09.2007 DE 102007041834
(71) Anmelder: Siemens Aktiengesellschaft, 80333 München (DE)
(72) Erfinder: Fehre, Jens, 91353, Hausen (DE); Nanke, Ralf, Dr., 91077, Neunkirchen am Brand (DE); Stetter, Martin, Dr., 80997, München (DE)

(57) **Zusammenfassung**

Die Erfindung betrifft ein Arzneimittel zur Behandlung eines Karzinoms, welches über die Blutbahn dem Karzinom zuführbar ist und zwei aneinander gekoppelte Wirkkomponenten (I, II) enthält, wobei
- die erste Wirkkomponente (I) von wenigstens einem Koppelmolekül (3) gebildet ist, das an ein vom Krebsgewebe (1) ausgebildetes Zielmolekül (8) spezifisch bindet, und
- die zweite Wirkkomponente (II) von wenigstens einem entzündungstypischen Signalmolekül (4) oder von wenigstens einem ein solches Molekül codierenden Stamm-Molekül gebildet ist.

## Beschreibung

Die Erfindung betrifft ein Arzneimittel zur Behandlung eines Karzinoms, insbesondere des Prostatakarzinoms. Bei Entzündungen werden in das Körpergewebe eingedrungene Fremdzellen oder Fremdkörper vor allem von Leukozyten angegriffen und zerstört bzw. unschädlich gemacht. Dagegen haben Karzinome die unangenehme Eigenschaft, dass sie vom Immunsystem des Körpers nicht bekämpft werden, so dass sie vielfach erst dann bemerkt werden, wenn eine erfolgreiche Behandlung etwa wegen Metastasenbildung kaum noch möglich ist.

Aufgabe der Erfindung ist es, ein Arzneimittel vorzuschlagen, welches das Immunsystem des Körpers zur Bekämpfung von Krebszellen anregt.

Diese Aufgabe wird durch ein Arzneimittel nach Anspruch 1 gelöst. Dieses ist über die Blutbahn zuführbar, also beispielsweise intravenös, oral oder rektal verabreichbar und enthält eine erste und zweite Wirkkomponente, die aneinander gekoppelt sind. Die Kopplung kann beispielsweise durch eine unmittelbare chemische Verbindung oder über eine mittelbare Verbindung erfolgen, indem etwa die Wirkkomponenten an einem Träger immobilisiert, etwa von diesem umhüllt oder daran chemisch oder auf sonstige Weise gebunden sind. Die erste Wirkkomponente ist von wenigstens einem im folgenden mit Koppelmolekül bezeichneten Molekül gebildet, das an ein vom Krebsgewebe ausgebildetes Zielmolekül spezifisch bindet. Die zweite Wirkkomponente dagegen ist von wenigstens einem entzündungstypischen Signalmolekül oder von wenigstens einem zu einem solchen Molekül umformbaren Stamm-Molekül gebildet. Aufgrund der Koppelmoleküle der ersten Wirkkomponente reichert sich das Arzneimittel selektiv im vaskulären Endothel des Krebsgewebes an. Aufgrund der entzündungstypischen Signalmoleküle der zweiten Wirkkomponente präsentiert sich das Krebsgewebe wie ein Entzündungsherd, der durch das Immunsystem des Körpers angegriffen wird. Es kommt, wie weiter unten noch genauer erläutert wird, zu einer Festsetzung von Leukozyten an den Signalmolekülen. Dabei besteht eine relativ hohe Wahrscheinlichkeit, dass zumindest ein Teil der Leukozyten über die Gefäßwand ins Krebsgewebe migriert, wodurch die entzündungsspezifischen Signal- bzw. Reaktionskaskaden ausgelöst und zumindest ein Teil der Krebszellen angegriffen und abgetötet werden. Das erfindungsgemäße Arzneimittel bewirkt also eine gezielte Anregung der Selbstheilungskräfte des Körpers.

Weitere Vorteile insbesondere auch der in Unteransprüchen angegebenen Abwandlungen gehen aus der folgenden Beschreibung hervor, welche auf die beigefügten Zeichnungen Bezug nimmt. Es zeigen jeweils in Form von schematischen Prinzipdarstellungen:
Fig. 1 das selektive Anreichern eines Arzneimittels im vaskulären Endothel eines Krebsgewebes,
Fig.2 und 3, die Wirkungsweise eines Arzneimittels, dessen zweite Wirkkomponente als Stamm-Molekül ein Plasmid enthält.

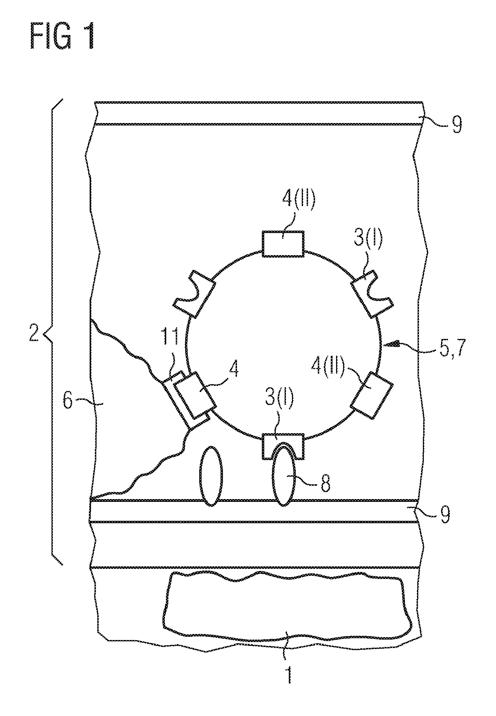

In den Abbildungen ist jeweils ein Krebsgewebe 1, beispielsweise ein Prostakarzinom, und ein dieses durchsetzendes oder diesem direkt benachbartes Blutgefäß 2 angedeutet. Bei dem Ausführungsbeispiel gem. Fig. 1 wird dem Krebsgewebe 1 über die Blutbahn ein Arzneimittel zugeführt, das eine erste Wirkkomponente I, die aus mehreren Koppelmolekülen 3 gebildet ist, und eine zweite Wirkkomponente II, die aus mehreren entzündungsspezifischen Signalmolekülen 4 gebildet ist. Als weitere Komponente ist ein Koppel- und Signalmoleküle zusammenhaltender Träger 5 vorhanden. Die Ausgestaltung des Trägers 5 ist prinzipiell beliebig. Es kann sich beispielsweise um ein Protein handeln, an das jeweils wenigstens ein Koppelmolekül 3 und wenigstens ein Signalmolekül 4 gebunden ist. Vorzugsweise werden als Träger 5 jedoch Microbubbles 7 verwendet. Das sind mikroskopisch kleine Hohlkugeln, deren Hülle beispielsweise aus einer Lipid-Doppelmembran gebildet ist, wobei die gegenseitige Bindung von Koppel- und Signalmolekülen 4 erreicht wird, indem die genannten Moleküle innerhalb des Microbubbles 7 vorhanden, in dessen Hülle inkorporiert und/oder an deren Außenseite gebunden sind. In dem in Fig. 1 gezeigten Fall sind die Koppel- und die Signalmoleküle 3,4 mit der Außenseite des Microbubble 7 verbunden.

Bei den Koppelmolekülen 3 handelt es sich um solche Moleküle oder Molekülstrukturen, die an Zielmoleküle 8 binden, die sich im Endothel 9 eines Blutgefäßes 2 ausbilden. Im Vorstadium des Karzinoms hat dieses noch keine Blutgefäße 2 entwickelt. Die Zielmoleküle 8 sind in diesem Fall im Endothel dem Krebsgewebe 1 direkt benachbarter Blutgefäße 2. Im Vorstadium der hochgradigen intraepithelialen Neoplasie etwa der Prostata (hgPIN) wird beispielsweise das CEACAM-1-Molekül (carzinoembryonic antigen-related cell adhesion molecule) ausgebildet. Daran spezifisch bindende Koppelmoleküle 3 sind beispielsweise CEACAM-1-Antikörper. Weiter fortgeschrittene Karzinome regen die Angiogenese, die Bildung von Blutgefäß 2 an. Die Angiogenese begleitende Wachstumsfaktoren wie beispielsweise VEGF (vasco endothelial growth factor) oder Alpha(V)-beta(3)- Integrin dienen dabei als Zielmoleküle 8, mit denen entsprechende Koppelmoleküle 3 als Bindungspartner, im genannten Fall etwa VEGF- bzw. Alpha(V)-beta(3)-Integrin-Liganden oder jeweils entsprechende Antikörper, zusammenwirken. Damit sowohl Karzinome im Vorstadium als auch solche, die sich bereits im Stadium der Angiogenese befinden, erkannt und behandelt werden können, kann das Arzneimittel die jeweils spezifisch bindenden Koppelmoleküle 3 in Kombination enthalten.

In den genannten Fällen anwendbare Koppelmoleküle 3 sind weiterhin sog. Aptamere. Es handelt sich dabei um kurze, stabile und spezifisch bindende RNA-Ketten. Schließlich sind als Koppelmoleküle 3 Anticaline geeignet. Dies sind einzelne Polypeptidketten mit ca. 180 Aminosäuren, die ähnlich spezifische Bindungseigenschaften aufweisen wie Antikörper, die aber leichter herstellbar sind.

Die zweite Wirkkomponente eines erfindungsgemäßen Arzneimittels hat, wie bereits erwähnt, die Aufgabe, dem körpereigenen Abwehrsystem vorzutäuschen, bei dem Krebsgewebe handele es sich um einen Entzündungsherd. Dieser Zielsetzung entsprechend wird die zweite Wirkkomponente II von wenigstens einem entzündungstypischen Signalmolekül 4 gebildet. Ein Entzündungsprozess läuft über mehrere verschiedene Stufen ab, denen jeweils eigene Signalmoleküle zugeordnet sind. Nicht alle dieser Signalmoleküle sind jedoch zu dem vorgesehenen Zweck geeignet. Das Anlocken und das Ansammeln von Leukozyten im Bereich eines Entzündungsherdes unterteilt sich in die Phasen der Anbindung (tethering), Rollen, gegebenenfalls Aktivierung, feste Adhäsion und transendotheliale Migration. Für jede Phase ist ein eigenes Paar aus Adhäsionsmolekül und daran bindendem leukozytenständigen Liganden verantwortlich. Die anfängliche Anbindung und das Rollen der Leukozyten kommt dadurch zu Stande, dass CLA-Moleküle (cutaneous lymphocyteassociated Antigen-Moleküle) in der Leukozyten-Membran an E-Selektin in der Endothelmembran kurzzeitig binden. Dadurch werden die Leukozyten aus dem Blutstrom um einen Faktor von ca. 100 abgebremst. Im nächsten Schritt werden Leukozyten durch Chemokine aktiviert und dadurch in die Lage versetzt, mit Hilfe von leukozytenständigen Integrinen an VCAM-1 Moleküle (vascular cytokine activated adhesion Moleküle) anzubinden. Es erfolgt zunächst eine weitere Adhäsion. Als Folge kann LFA-1 (leukocyte function antigen) des Leukozyten an I-CAM-1 (intercellular cytokine-activated adhesion molecule) im Endothel anbinden, wodurch der Leukozyt immobilisiert wird. Durch die Immobilisierung werden sowohl im Endothel als auch im Leukozyten Signalkaskaden ausgelöst, die schließlich das Einwandern des Leukozyten in das betroffene Gewebe gefolgt von einer Entzündungsreaktion bewirken. Die Bindung zwischen VCAM-1 und leukozytenständigen Integrinen (beispielsweise dem alpha-(4)-beta (1) Integrin der Haut) gefolgt von der Bindung zwischen ICAM-1 und leukozytenständigem LFA-1 stellen Schlüsselprozesse bei vielen verschiedenen Gewebearten dar. Diese eignen sich daher besonders zum Erreichen des erfindungsgemäßen Ziels, nämlich dem Immunsystem vorzutäuschen, bei dem Krebsgewebe handele es sich um einen Entzündungsherd. Als entzündungsspezifische Signalmoleküle 4 werden daher Cytokinaktivierte Adhäsionsmoleküle (CAM), vorzugsweise VCAM-1 und ICAM-1 eingesetzt. Diese sind bei dem Ausführungsbeispiel gem. Fig. 1 mit der Außenseite des Microbubbles 7 (oder mit einem auf sonstige Weise ausgestalten Träger 5)verbunden. Wie oben erwähnt, werden die Leukozyten durch Chemokine aktiviert und dadurch in die Lage versetzt, mit Hilfe von leukozytenständigen Integrinen an VCA-1-Moleküle zu binden. Zur Erhöhung der Wirksamkeit des vorgeschlagenen Arzneimittels ist es daher zweckmäßig, als dritte Wirkkomponente wenigstens ein Chemokin der genannten Art beizufügen. Hinsichtlich der Wirksamkeit, also der sicheren Auslösung der entzündungsspezifischen Reaktionen, ist es vorteilhaft, wenn das Arzneimittel sowohl VCAM-1 als auch ICAM-1 als Signalmoleküle 4 enthält.

Bei dem in Fig.1 gezeigten Ausführungsbeispiel bindet ein Leukozyt 6 mit einem Liganden 11 an das Signalmolekül 4 des Trägers 5 an, was den Gegebenheiten bei tatsächlichen Entzündungsvorgängen nicht ganz entspricht, da hier die Signalmoleküle in einem vaskulären Endothel 9 lokalisiert sind. Um den tatsächliche Verhältnissen näher zu kommen und damit die Wahrscheinlichkeit der Migration eines Leukozyten 6 in Endothelzellen und das Auslösen entzündungsspezifischer Signalkaskaden zu erhöhen ist bei einer Variante des Arzneimittels vorgesehen, Microbubbles 7 als Träger 5 zu verwenden, die durch Beaufschlagung mit Ultraschall, beispielsweise über eine Rektalsonde, zerstörbar sind. Bei der Zerstörung der Microbubbles 7 werden die in oder an ihnen vorhandenen Signalmoleküle freigesetzt. Mit der Ultraschallbeaufschlagung ist außerdem der als Sonoporation bekannte Effekt verbunden, dass das Endothel vorübergehend destabilisiert wird. Dabei bilden sich in den Zellmembranen Poren, in die die Signalmoleküle teilweise eindringen, so dass sie nach der Ultraschallbehandlung wie bei normalen Entzündungsherden im Endothel verankert sind.

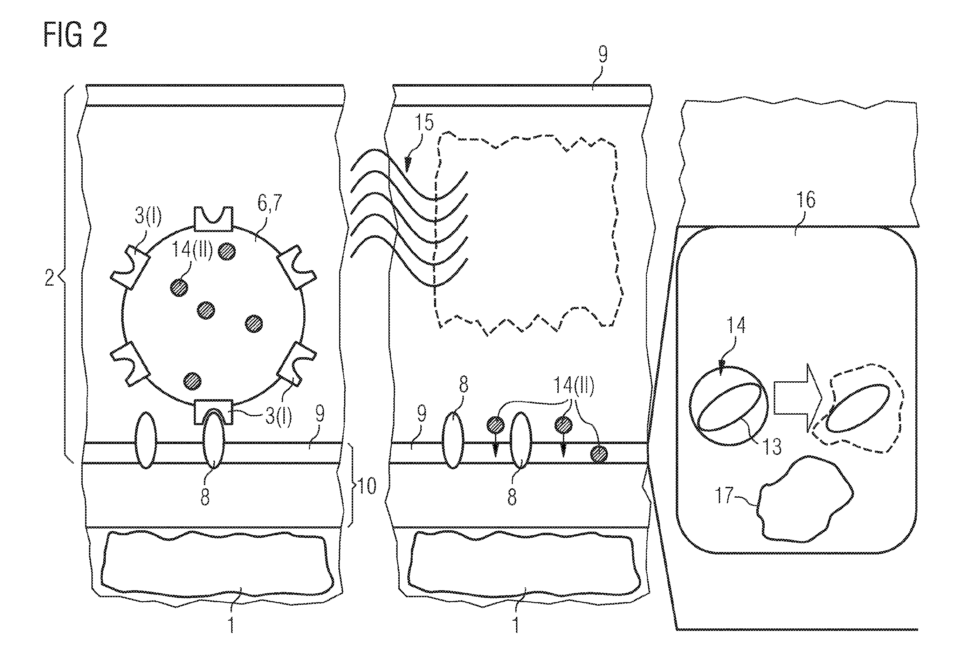

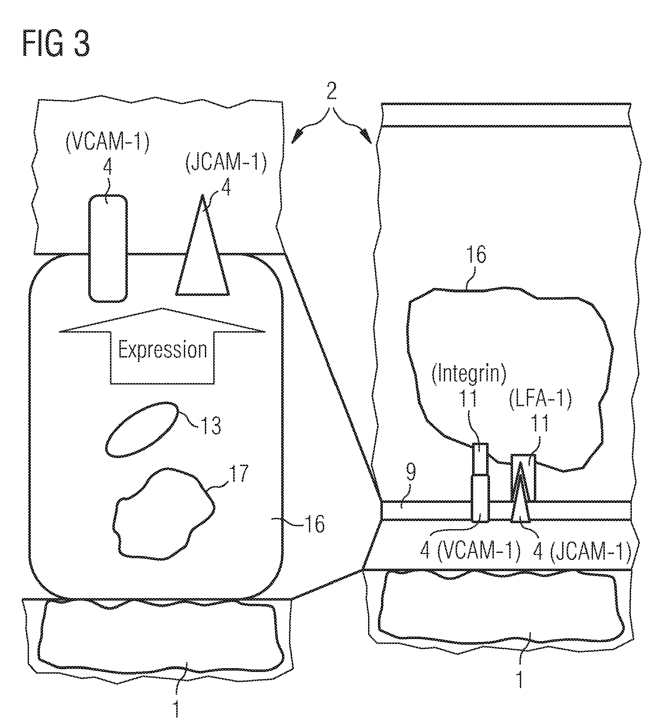

Bei einer besonders bevorzugten Variante des Arzneimittels werden die Signalmoleküle 4 nicht als solche, sondern in Form eines sie codierenden Stamm-Moleküls, insbesondere eines Plasmids zum Zielgewebe transportiert. Das eigentliche Zielmolekül wird erst am Zielort und zwar innerhalb einer Endothelzelle gebildet. Das Einbringen des Stamm-Moleküls bzw. des Plasmids in die Endothelzellen erfolgt durch transiente Transfektion, was in Fig. 2 und 3 dargestellt ist. Ein als Träger 5 fungierendes Microbubble 7 trägt an seiner Oberfläche Koppelmoleküle 3, beispielsweise CEACAM-1-Liganden. Diese koppeln an Zielmoleküle 8, beispielsweise CEACAM-1- Moleküle an, wodurch das Microbubble 7 am Endothel 9 immobilisiert wird. Innerhalb des Microbubbles 7 befinden sich die zweite Wirkkomponente II, die ein beispielsweise CAM (cell adhesion molecule) kodierendes Plasmid 13 enthält. Als Transportvehikel zur Übertragung des Plasmids in eine Endothelzelle dient ein viraler Vektor, d.h. ein für den Menschen unschädlicher Virus 14. Durch Einwirkung von Ultraschallwellen 15 wird die Hülle des Microbubbles 7 zerstört und somit die Viren 14 freigesetzt. Gegebenenfalls unterstützt durch den Effekt der Sonoporation dringen die Viren in das Endothel 9 bzw. in Endothelzellen 16 ein. Dort wird das Plasmid 13 freigesetzt. Wie bei viralen Erkrankungen auch, liest die Wirtszelle, genauer der Zellkern 17, die Plasmiden-DNA ab und exprimiert die darauf kodierten Gene. Es werden also die entsprechenden Proteine, im vorliegenden Fall ICAM- und/oder VCAM-Moleküle gebildet. Im Gegensatz zu einer externen Zuführung von CAM-Molekülen oder sonstigen entzündungsspezifischen Signalmolekülen 4 steht auf Grund der ständigen Nachbildung in Folge Genexpression eine wesentlich größere Anzahl von Signalmolekülen 4 zur Verfügung, welche in das vaskuläre Endothel 9 inkorporiert werden. Die vom Immunsystem ausgelöste Reaktion, beginnend mit der Immobilisierung von Leukozyten im Krebsgewebe findet daher in einem entsprechend starken Ausmaß statt. Beispielsweise wird ein Plasmid 13 in die Endothelzellen 16 eingeschleust, welches als entzündungstypische Signalmoleküle sowohl VCAM-1 als auch ICAM-1 exprimieren. An die genannten Signalmoleküle 4 koppelt ein Leukozyt 17 mit einem Integrin- bzw. einem LFA-1-Liganden 11 an. Neben entzündungstypischen Signalmolekülen 4 kann auf dem Plasmid 13 auch die genetische Information für andere Proteine, beispielsweise für Chemokine vorhanden sein, welche die Leukozyten 17 aktivieren und dadurch in die Lage versetzen, mit Hilfe von leukozytenständigen Integrinen an VCAM-1-Moleküle anzubinden. Die oben erwähnte Zerstörung der Microbubbles 7 mit Hilfe von Ultraschall ist nicht unbedingt erforderlich. Es können auch solche Microbubbles 7 eingesetzt werden, die sich im Blutserum selbst auflösen. In diesem Fall kann eine retardierende Umhüllung zweckmäßig sein, die bei einer oralen, rektalen oder intravenösen Verabreichung zumindest so lange Bestand hat, bis eine Immobilisierung in den Blutgefäßen 2 des Krebsgewebes 1 bzw. in diesem benachbarten Blutgefäßen stattgefunden hat.

## Patentansprüche

1. Arzneimittel zur Behandlung eines Karzinoms, welches über die Blutbahn dem Karzinom zuführbar ist und zwei aneinander gekoppelte Wirkkomponenten (I, II) enthält, wobei
- die erste Wirkkomponente (I) von wenigstens einem Koppelmolekül (3) gebildet ist, das an ein vom Krebsgewebe (1) ausgebildetes Zielmolekül (8) spezifisch bindet, und
- die zweite Wirkkomponente (II) von wenigstens einem entzündungstypischen Signalmolekül (4) oder von wenigstens einem ein solches Molekül codierenden Stamm-Molekül gebildet ist.

2. Arzneimittel nach Anspruch 1, bei dem die erste Wirkkomponente (I) ein Koppelmolekül (3) umfasst, das an ein in einem Vorstadium des Karzinoms ausgebildetes Zielmolekül (8) bindet.

3. Arzneimittel nach Anspruch 1, bei dem die erste Wirkkomponente (I) ein Koppelmolekül (3) umfasst, das an ein im Stadium der Angiogenese ausgebildetes Zielmolekül (8) bindet.

4. Arzneimittel nach Anspruch 1, bei dem die erste Wirkkomponente (I) ein an ein in einem Vorstadium des Karzinoms ausgebildetes Zielmolekül (8) und ein an ein im Stadium der Angiogenese ausgebildetes Zielmolekül (8) bindendes Koppelmolekül (3) umfasst.

5. Arzneimittel nach Anspruch 2 oder 4, mit einem Koppelmolekül (3), das an ein in der hochgradigen intraepithelialen Neoplasie ausgebildeten Zielmolekül (8) bindet.

6. Arzneimittel nach Anspruch 2, 4 oder 5, mit einem an CEA-CAM1 als Zielmolekül (8) bindenden Koppelmolekül (3).

7. Arzneimittel nach Anspruch 6, mit einem CEACAM1-Antikörper als Koppelmolekül (3).

8. Arzneimittel nach Anspruch 3 oder 4, mit einem Koppelmolekül (3), das an den Wachstumsfaktor VEGF bindet.

9. Arzneimittel nach Anspruch 8, mit einem VEGF-Liganden oder
- Antikörper als Koppelmolekül (3).

10. Arzneimittel nach Anspruch 3 oder 4 mit einem Koppelmolekül (3), das an Integrin bindet.

11. Arzneimittel nach Anspruch 10, mit einem Koppelmolekül, das an Alpha(V)beta(3)-Integrin bindet.

12. Arzneimittel nach Anspruch 10 oder 11, mit einem Integrin-Liganden oder - Antikörper als Koppelmolekül (3).

13. Arzneimittel nach einem der vorhergehenden Ansprüche, mit wenigstens einem Koppelmolekül (3) aus der Gruppe Anticaline und Aptamere.

14. Arzneimittel nach einem der vorhergehenden Ansprüche, bei dem die zweite Wirkkomponente (II) ein Cytokin-aktiviertes Adhäsionsmolekül (CAM) umfasst.

15. Arzneimittel nach Anspruch 14, das als zweite Wirkkomponente (II) VCAM-1 und/oder ICAM-1 enthält.

16. Arzneimittel nach einem der vorhergehenden Ansprüche, das als dritte Wirkkomponente wenigstens eine die Adhäsion von Leukozyten (6) an entzündungsspezifischen Signalmolekülen (8) aktivierende Substanz enthält.

17. Arzneimittel nach Anspruch 16, das als dritte Wirkkomponente wenigstens ein Chemokin enthält.

18. Arzneimittel nach einem der Ansprüche 1 bis 13, bei dem die zweite Wirkkomponente (II) als Stamm-Molekül ein den genetischen Code für wenigstens ein entzündungsspezifisches Signalmolekül (8) umfassendes Plasmid (13) enthält.

19. Arzneimittel nach Anspruch 18, das als dritte Wirkkomponente wenigstens ein Chemokin enthält.

20. Arzneimittel nach Anspruch 18, das ein den genetischen Code für wenigstens ein Chemokin umfassendes Plasmid (13) enthält.

21. Arzneimittel nach Anspruch 18, 19 oder 20, mit einem ein Plasmid (13) enthaltenden Virus (14).

22. Arzneimittel nach einem der vorhergehenden Ansprüche, bei dem die Wirkkomponenten mit Microbubbles (7) als Träger (5) verbunden sind, welche mit Hilfe von Ultraschall zerstörbar sind.
